Europäisches Patentamt

(19) European Patent Office

Office européen des brevets

(11) Publication number: **0 231 236 B1**

(12) **EUROPEAN PATENT SPECIFICATION**

(45) Date of publication of patent specification :
09.10.91 Bulletin 91/41

(51) Int. Cl.⁵ : **C07K 3/20, // A61K37/14**

(21) Application number : 86904259.8

(22) Date of filing : 26.06.86

(86) International application number :
PCT/GB86/00374

(87) International publication number :
WO 87/00177 15.01.87 Gazette 87/01

(54) **PURIFICATION OF HEMOGLOBIN AND MODIFIED HEMOGLOBIN BY AFFINITY CHROMATOGRAPHY.**

(30) Priority : 26.06.85 CA 485392

(43) Date of publication of application :
12.08.87 Bulletin 87/33

(45) Publication of the grant of the patent :
09.10.91 Bulletin 91/41

(84) Designated Contracting States :
AT BE CH DE FR LI LU NL SE

(56) References cited :
DD-A- 149 861
Chemical Abstracts, vol. 79, no. 5, 06 Aug. 1973, Columbus, Ohio, US; R. Lamed et al.: "Covalent coupling of nucleotides to agarose for affinity chromatography", see p. 217, abstract 29183d .
Nature, vol. 221, 15 Febr. 1969; R. Benesch et al.: "Intracellular organic phosphates as regulators of oxygen release by heamoglobin", pp 618-622, see p 618, abstract,col. 1, 2nd para., p. 621,col 1, 4th para. .
Chemical Abstract, vol. 85, no. 13, 27 Sept 1976, Columbus, Ohio, US R. Benesch et al.: " The allosteric effect of inositol hexasulfate on oxygen binding by hemoglobin", see p. 181, abstract 88796c .
Chemical Abstracts, vol. 83, no. 1, 07 July 1975, Columbus, Ohio, U.S. P.H. CHAN et al.: " One step purification of D-galactose and L-arabinose kinases from Phaseolus aureus seedlings by ATP-sepharose", see page 302, abstract 3301y .

(56) References cited :
Chemical Abstracts, vol. 79, no. 7, 20 Aug.73, Columbus, Ohio, US M.KLEIN et al.: "Affinity chromatography. Specific binding of hemoglobin on agarose-linked haptoglobin" see p. 140, abstract 39807z .
Journal of Chromatography, Biomedical Applications, vol. 374, no.1, 10 Jan 1986 J.C. HSIA et al.: "Purification of stroma-free haemoglobin by ATP-agarose affinity chromatography", pp 143-148, see pp 143, 146-148 .

(73) Proprietor : HEMOSOL INC.
6850 Goreway Drive
Mississauga, Ontario, L4V 1P1 (CA)

(72) Inventor : HSIA, Jen, Chang
10 Cherry Hills Road
Concord, Ontario L4K 1M4 (CA)

(74) Representative : Jukes, Herbert Lewis et al
Swann, Elt & Company 31 Beaumont Street
Oxford OX1 2NP (GB)

## Description

The invention relates to hemoglobin-based acellular oxygen carriers, and more specifically to the purification of hemoglobin and modified hemoglobin by the technique of affinity chromatography.

Research in the development of an acellular oxygen-carrying solution for use as a blood substitute has concentrated in two areas, namely solutions of hemoglobin-based and perfluorocarbon substances, as described for example by Biro (Can. Med. Assoc. J., Vol. 129, 1983, pp. 237-244). Recent work has indicated that the perfluorocarbons are less suitable due, for example, to the special clinical conditions needed for their use and to problems of toxicity. Present research is, therefore, focussed on potential hemoglobin-based blood, substitutes. Since hemoglobin in solution (instead of in the red blood cell or corpuscle where it is naturally found) does not act as a physiologically satisfactory oxygen carrier, the development of hemoglobin derivatives with good oxygen-carrying and circulatory characteristics is warranted.

The principal source of hemoglobin for this research is presently "outdated" donor blood. Red blood cells are naturally rich in hemoglobin. Isolation of hemoglobin from this source has been accomplished by a number of methods involving the separation of hemoglobin from other red cell cytoplasmic proteins and from the cell stroma.

The standard technique for purification of hemoglobin is ion-exchange chromatography. This technique has been widely used in analytical and diagnostic applications (see, for example, Benesch and Benesch, Meth. Enzymol., Vol. 76, pp. 174-179, 1981; and Hsia et al, J. Chromatog., Vol. 303, pp. 425-428, 1984).

A much more rapid and specific purification technique, however, is affinity chromatography, which selectively isolates biomolecules by virtue of their binding specificity for particular molecules, radicals, ions, or groups called ligands, which form complexes with the biomolecules. Generally, in this technique, the ligand is immobilized on a chromatographic gel, and a solution containing the molecule of interest is passed through the gel. The molecule of interest is retained in the gel by virtue of its specific binding to the ligand, while the other components present in this solution are eluted.

It is known that hemoglobin specifically binds small polyanionic molecules, especially 2,3-diphosphoglycerate (DPG) and adenosine triphosphate (ATP), naturally present in the mammalian red cell (Benesch and Benesch, Nature, Vol. 221, p. 618, 1969). The hemoglobin site at which this binding takes place is located at or near the centre of the tetrameric structure of hemoglobin (Arkone, A., Nature, Vol. 237, p. 146, 1972). The binding of those polyanionic molecules to the hemoglobin in site is important in regulating the oxygen-binding affinity, since this binding of the polyanionic molecules allosterically affects the conformation of hemoglobin leading to a decrease in such oxygen-binding affinity (Benesch and Benesch, Biochem. Biophys. Res. Comm., Vol. 26, p. 162, 1967). Conversely, the binding of oxygen to hemoglobin allosterically reduces the affinity of hemoglobin for the polyanion. Oxyhemoglobin, therefore, binds DPG and ATP weakly. This is shown, for example, by studies of spin-labelled ATP binding to oxy- and deoxyhemoglobin as described by Ogata and McConnell (Ann. N.Y. Acad. 5c., Vol. 222, p. 56, 1973).

More specifically, the aforementioned binding site for the polyanionic molecules is located generally between B-globin chains of the hemoglobin, and can be regarded as a "cleft" between the chains. Under natural conditions in the red blood cell, this cleft is occupied by a molecule of DPG (in its aldehydic form). When the hemoglobin binds with oxygen, to form oxyhemoglobin, a conformational structural rearrangement takes place which results in the expulsion of the DPG from the cleft and is believed to cause the effective closing of the cleft. The binding of one molecule of oxygen to the tetrameric hemoglobin actually increases its oxygen affinity, for binding to a second, third and fourth molecule of oxygen.

Previous studies of hemoglobin and attempts to prepare cell free solutions of hemoglobin for use as blood substitutes have shown that a wide variety of polyanionic groups and molecules will bind in the binding site or cleft naturally occupied by DPG. Some of these studies have been part of attempts to provide means for binding together the B-chains of tetrameric hemoglobin in solution to counteract the undesirable tendency of the tetrameric hemoglobin to dissociate into dimers. In the natural hemoglobin in the blood cell, this function is fulfilled at least in part by the DPG in the cleft, but the DPG tends to diffuse out in solution. Among the substances which will bind in the cleft as "DPG substitutes" are various organic phosphates, diphosphates and polyphosphates, for example pyridoxal phosphate; phosphates or nucleic acids such as ADP, ATP, guanosine phosphates, cytosine phosphates, thymine phosphates, uracil phosphates, etc.; inositol phosphates; carbohydrate phosphates and sulfates; carbohydrate mono- and poly-carboxylates etc. A wide variety of such binding substances is known to those skilled in the art, and published in the relevant scientific literature.

Because oxygenation of hemoglobin to form oxyhemoglobin effectively deactivates the polyanionic binding site ("closes the cleft") of hemoglobin, it has been necessary, according to prior art, in order to exploit the polyanion-binding specificity of hemoglobin, or indeed to perform any adjustment of its oxygen-binding affinity by chemically modifying the polyanion binding site, that the hemoglobin be deoxygenated. However, hemoglobin as it exists in solutions or mixtures exposed to air is in its oxy state, i.e. (oxy)hemoglobin. In fact, it is difficult to maintain hemoglobin solutions in the unoxygenated state, throughout procedures which involve contact of the solutions with air, e.g. a chromatographic procedure. Because of these difficulties, the technique of affinity chromatography has not been used in the prior art to purify hemoglobin.

Unexpectedly, it has now been found that (oxy)hemoglobin may be isolated and purified by applying the technique of affinity chromatography. The technique described is, of course, applicable to isolation and purification of unoxygenated hemoglobin.

The invention comprises the use of affinity chromatography for the isolation and purification of (oxy)hemoglobin from whole blood or other sources or mixtures, by virtue of reversible binding of hemoglobin via its polyanion binding site to a polyanion immobilized on a chromatographic gel support. This discovery permits the purification of hemoglobin in that state in which it predominates under normal laboratory conditions.

According to the invention, a method for the isolation of (oxy)hemoglobin from (oxy)hemoglobin-containing solutions or mixtures is provided, comprising:

(a) immobilizing a polyanion which specifically binds hemoglobin via its polyanion binding site, on chromatographic gel; and

(b) passing the (oxy)hemoglobin-containing solution or mixture through the gel, whereby the (oxy)hemoglobin is retained in the gel, while other components of the solution or mixture are eluted.

More specifically, if (oxy)hemoglobin solutions are passed through ATP-agarose gels (commercially available or prepared by known methods), it is observed that hemoglobin is retained by the gel, resulting in a so-called "red gel". The oxygen-binding characteristics of hemoglobin in the red gels, as demonstrated by the oxygen dissociation curve, are similar to those of its soluble ATP complex ($P_{50}$ - 35 mmHg) rather than of hemoglobin in Bis-Tris buffer solution ($P_{50}$ - 7 mmHg). Hemoglobin in the red gel is, therefore, allosterically modified by ATP binding to the polyanion binding site. As further evidence of this, the introduction of a solution containing any of a variety of competing anions causes elution of hemoglobin from the gel. Some of these competing anions, in order of decreasing effectiveness, are inositol hexaphosphate > ATP ≈ pyridoxal phosphate ≈ DPG > adenosine diphosphate > phosphate ion > chloride ion. This specific and reversible binding, in addition to the oxygen dissociation kinetics of the red gel, represent a functional demonstration of the specific binding of (oxy)hemoglobin to the polyanion affinity gel.

This finding, which contradicts literature reports of the mutually exclusive binding of oxygen and polyanions to (oxy)hemoglobin, is attributed to the following. Firstly, the ATP-agarose gel presents to the hemoglobin a high local concentration of ATP, stoichiometrically favouring binding. Secondly, it is believed that the hydrophobic spacer molecule which links ATP to the gel, and the agarose gel material itself, acts to enhance ATP binding to the polyanion binding site of hemoglobin. It is not, however, to be inferred that the invention is to be limited to any particular theory or mode of action, or that it is restricted to the specific use of agarose gels, ATP ligands and specific hydrophobic spacer molecules.

Thus, the technique of affinity chromatography can be applied to the isolation and purification of (oxy) hemoglobin. This represents an improvement upon conventional techniques in that it specifically isolates hemoglobin of high purity in a one-step chromatographic procedure, using gentle conditions which do not disrupt the native structure of the protein. It is readily adapted to large-batch preparations, as will be necessary for scaled-up production of hemoglobin-based blood substitutes. Finally, in terms of the functional quality of the product, this procedure selectively isolates hemoglobin with its ability to bind polyanions intact, giving functionally intact hemoglobin while at the same time eliminating undesirable contaminants.

Whilst the process of the invention is commonly practised by providing a column containing the chromatographic gel and flowing the (oxy)hemoglobin-containing solution through the column, followed by appropriate eluting reagents, this manner of operation is not essential. One can, if desired, mix the solution and gel in a closed vessel under agitation, separate the solids and liquids e.g. by filtration or centrifugation, and then repeat the operation with liquid elutant.

In the preferred form of this aspect of the present invention, (oxy)hemoglobin is isolated by affinity chromatography on affinity gels comprising a polyanionic-molecule linked by a hydrophobic spacer group (crosslinking agent) to a chromatographic gel support by known methods. Examples of polyanionic ligands are diphosphoglycerate, nucleoside phosphates, inositol phosphates and sulphates, etc. In fact, any polyanionic ligand, which is able to bind in the binding site or cleft naturally occupied by DPG in hemoglobin, may be used. A wide variety of these are known and published, as previously discussed.

An especially preferred ligand for use on the chromatographic gel, at least for laboratory purposes, is ATP. On a larger scale, however, ATP tends to present difficulties of purification. The ligand should not bind to the (oxy)hemoglobin so strongly that difficulties of elution of the (oxy)hemoglobin from the gel are encountered. If the (oxy)hemoglobin is too tightly bound, then side reactions may occur in the use of the necessarily strong reagents for elution, e.g. protein denaturing. When using organic polyphosphates, the bond strength of,the ligand to the (oxy)hemoglobin appears to increase with increasing numbers of phosphate groups. The optimum bond strength appears to be derived from tri-, tetra- and penta-phosphates, so that preferred ligand-forming compounds are nucleoside tri- and tetra-phosphates, such as ATP, inositol tetraphosphate, inositol penta-phosphate and the like, including mixtures thereof. The ligand molecule is chemically bonded to the gel side groups in a manner such that the phosphate, sulphate or carboxylate functional groups are left intact to react with the polyanion binding site or "cleft" in the hemoglobin. In the case of ATP, it can be bound to the gel via its N-6 position or 8-position of adenosine, or through the periodate oxidized ribose moiety, for example.

The chromatographic gel is provided with a crosslinking agent or spacer, which is effectively a chemical side chain group covalently linked to the gel at one end and providing a reactive group on the other end for chemical attachment to the ligand compound. While these spacers do not need to be linear chemical groups, they should provide a spacing between the gel backbone and the reactive group for ligand attachment of at least about 5Å, and preferably at least 6Å. In practice, this means that they should contain a minimum of four linearly arranged atoms separating the gel from the functional group. Otherwise, insufficient loading of ligand groups onto the chromatographic gel might occur. The spacer groups should be hydrophobic, so that they are not broken during chromatographic processes using aqueous solutions. They should be stable and inert to all the components of the mixture to be separated, and to the elution reagents to be used. Examples of suitable reagents to be used to react with the gels to provide suitable spacer groups are adipic acid, diamino-hexane and derivatives thereof, such as adipic dihydrazide, and various other, known diacids and diamino compounds.

The choice of suitable chromatographic gel is within the skill of the art, and can be made from various commercially available products, provided that certain basic criteria are observed. The gel needs to be substantially water insoluble, derivatizable and non-toxic. It needs to be able to tolerate the regeneration conditions and processes of treatment, for meeting sterile, sanitary transfusion requirements. Thus it must be readily sterilizable, without impairment of its chemical properties.

Examples of chromatographic gel supports are agarose and silica gels.

In general, modified cross-linked polysaccharide chromatographic gels are useful, as exemplified by those commercially available from Pharmacia A.S. under the trade names Sephadex, Sepharase, Sepharyl and Sepharose. Commercially available chromatographic silica gels and modified silica gels are also suitable. The gels in many cases are available with spacer functional groups already attached. They can be obtained with functional amino, epoxy or hydroxy groups. They may also, in addition, have an appropriate ligand chemically attached to the spacer group, so they are effectively ready for use in the process of the present invention.

The chromatographic procedure may be conducted as follows. A solution containing (oxy)hemoglobin and other components, e.g. red cell cytoplasmic proteins, in equilibrium with air, is injected into the affinity gel and eluted under conditions which favour hemoglobin binding to the gel, according to the known practices of affinity chromatography. Ultimately, the non-hemoglobin components are eluted from the gel and hemoglobin is retained, causing the gel to appear red. Conditions are then changed to favour dissociation of hemoglobin from the gel, and it is eluted as a pure hemoglobin fraction. The preferred condition for elution of hemoglobin from the gel is the use of buffer containing an anion which competes with the polyanionic moiety of the affinity gel for specific binding to hemoglobin, thus displacing it from the gel. The buffer is one which does not affect the binding of hemoglobin to the polyanion moiety. Bis-Tris buffer, pH 7.0 has been found suitable.

According to another aspect of the invention, applicant's hemoglobin purification technique can be applied to the removal of residual unmodified hemoglobin from liquid reaction mixtures containing modified and unmodified hemoglobin following chemical modification of the hemoglobin to improve its oxygen-carrying and circulatory characteristics. The reactions involved in modifying hemoglobin are typically incomplete and result in a mixture of modified and unmodified hemoglobin fractions. Residual unmodified hemoglobin poses problems in vivo due to excessive oxygen-binding affinity, its rapid excretion from the circulation, and possibly vasoconstrictor activity. Although this unmodified hemoglobin is currently removed from modified hemoglobin containing reaction mixtures on an analytical scale by ion-exchange chromatography, it is generally not removed in large scale preparations. Without removal of substantially all the unmodified hemoglobin, the product is not acceptable for human use.

It will be apparent hereinafter that applicant's technique is capable of removing residual unmodified hemoglobin from modification reaction mixtures on a preparative scale, this comprising an important quality-control step in the modification of hemoglobin as a starting material for the preparation of acellular oxygen-carrying solutions.

More specifically, modified hemoglobin is considered here to mean purified, acellular hemoglobin to whose polyanion binding site a DPG analogue has been co-valently attached by any of several known methods, or by any other method, for the purposes of (a) stabilizing the native tetrameric structure of hemoglobin in solution, and (b) allosterically lowering the oxygen-binding affinity of hemoglobin, simulating the regulation which is performed naturally in the red blood cell by DPG or its naturally occurring analogues, e.g. ATP, inositol pentaphosphate, etc.

The reaction mixture, comprising the product of the modification reaction, is assumed to contain modified hemoglobin and residual, unmodified hemoglobin which remains due to the incomplete nature of the modification reaction, with DPG analogues such as ATP, other nucleoside phosphates, inositol tri-, tetra- and pentaphosphates, pyridoxal phosphate, glyoxylic acid, etc.

The reaction mixture is passed through the polyanion affinity column according to the known procedures of affinity chromatography. In the unmodified hemoglobin molecule, the polyanion binding site is unoccupied and the molecule is, therefore, retained by the gel via specific binding of the polyanion moiety of the gel to this site. Conversely, in the modified hemoglobin molecule, the polyanion binding site is by definition occupied by the covalertly attached polyanion or other modifying agent. Modified hemoglobin, therefore, will not bind specifically to the gel, and is eluted as the unretained fraction.

Thus polyanion affinity chromatography is capable of readily separating hemoglobin from non-hemoglobin components in a mixture according to the first aspect of the invention, and is also capable of separating modified from unmodified hemoglobin, regardless of the modification procedure used, on the basis of the state of the polyanion binding site. This allows purification of hemoglobin, especially from unconventional sources where the use of conventional purification techniques is not feasible, and also allows preparation of an essentially pure modified hemoglobin fraction by adding one chromatographic step to any of several known modification procedures.

When the process of the present invention is applied to the separation of unmodified hemoglobin from a modified hemoglobin adapted for blood substitute use, e.g. ATP-hemoglobin, pyridoxal phosphate (PyP)-hemoglobin or glyoxylated (G)-hemoglobin, the resultant purified modified hemoglobin shows unexpected and unpredictable advantages. For example, the so-purified modified hemoglobin in use as a blood substitute shows a marked reduction in vasoconstrictive activity, a problem previously encountered persistently with such compounds. Moreover, the purified product exhibits an advantageously reduced oxygen affinity, as compared with such modified hemoglobins purified by other methods. An ATP-hemoglobin is thus obtained which has substantially similar oxygen binding properties to whole blood.

In the drawings which illustrate the preferred embodiments of the invention:

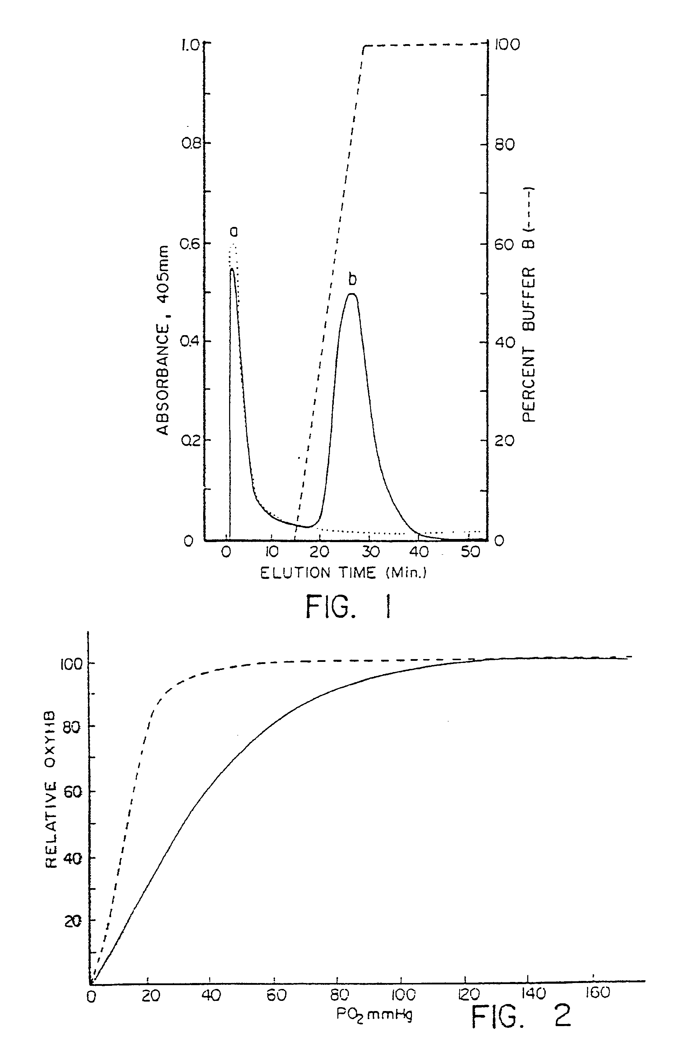

Figure 1 - Elution profile of hemoglobin chromatographed on an ATP-agarose affinity column.

Chromatogram of 10 ul (50 ug) of stroma-free hemoglobin (SFH) on an analytical capillary column packed with 10 ul of AGATP gel. Elution of SFH by buffer A (50 mM Bis-Tris buffer, pH 7.0) shows only peak "a", the unretained fraction (dotted line). Introduction of a gradient of buffer B (10 mM ATP in buffer A; gradient shown as broken line) results in the elution of a retained fraction, peak "b" (solid line). Experimental conditions were: flow rate 0.1 ml/min., temperature approximately 20°C, using a FPLC chromatographic system (Pharmacia Model LCC 500). The chromatographic gel is an agarose gel treated with CNBr and adipic dihydrazide to provide the chemical spacer group, with o-ATP as ligand. It is purchased commercially in this form, ready for use.

Figure 2 - Modification of the oxygen affinity of SFH by AGATP gel.

Oxygen dissociation curve of SFH in the presence of AGATP gel (solid line) and plain agarose gel (broken line) in 50 mM Bis-Tris buffer, pH 7.0, at 37°C. Curves were obtained using a Hem-O-Scan oxygen dissociation analyzer. The same chromatographic gel was used.

Figure 3 - Electrophoretic pattern on cellulose acetate of SFH before and after AGATP gel chromatography.

Electrophoresis was performed in barbital buffer, pH 8.8. Lanes 1 and 2 are the retained and unretained fractions respectively (see Figure 1). Lane 3 is a control sample of the starting SFH. Arrows "c" and "d" indicate unidentified minor components of SFH which are enriched in the unretained fraction. Electrophoresis on the Mylar-supported cellulose acetate was run at 200 V for 45 minutes and stained with Ponceau S.

## Example 1

Purification of Stroma-free Hemoglobin by Agarose-ATP Affinity Chromatography

In this example, the source of (oxy)hemoglobin is stroma-free hemoglobin. Agarose-adipic-adenosine-5'-triphosphate (AGATP) was prepared by the method of Lamed et al. (Biochem. Biophys. Acta, Vol. 304, p.231, 1973). Stroma-free hemoglobin (SFH) was prepared by the method of Rabiner et al. (J. Exp. Med,., Vol. 126, p. 1127, 1967). SFH was first dialyzed against 3 changes of 50 mM Bis-Tris buffer, pH 7.0 (Buffer A), and then 500 ul (20 mg) of the SFH solution was applied to a Pharmacia HR 5/5 column packed with approximately 1 ml of AGATP gel equilibrated with the same buffer. The column was washed with 10 bed volumes of buffer A at a flow rate of 0.5 ml/min. until the eluent was clear, and then buffer B (buffer A containing 1- mM ATP) was introduced via a linear gradient to elute the retained fraction. Both the retained and unretained fractions were collected, concentrated, and dialyzed against buffer A. Cellulose acetate electrophoresis in barbital buffer, pH 8.8 (High Resolution Buffer, Gelman Sciences) was done for each fraction using the starting SFH preparation as a control. For oxygen dissociation studies, a small amount of the red gel was resuspended in 2 ul of buffer A and its oxygen dissociation curve measured using a Hem-O-Scan oxygen dissociation analyzer. As a control, SFH mixed with agarose gel was suspended in solution and its oxygen dissociation curve similarly obtained.

Figure 1 shows the binding of hemoglobin to the AGATP gel in buffer A, and the selective gradient elution profile resulting from the addition of 10 mM ATP to buffer A. -This indicates that hemoglobin is specifically retained via binding of the ATP moiety of the gel to its polyanion binding site. As further evidence of this specificity, SFH is displaced from the gel by the following anions in order of decreasing effectiveness: inositol hexaphosphate > ATP $\simeq$ pyridoxal phosphate $\simeq$ diphosphoglycerate > adenosine diphosphate > phosphate ion > chloride ion (results not shown). Figure 2 shows a typical oxygen dissociation curve of hemoglobin bound to the AGATP gel. The hemoglobin-AGATP complex is shown to have a $P_{50}$ of 35 mmHg, similar to that of SFH in the presence of four molar equivalents of ATP. By contrast, the control SFH-agarose mixture has a $P_{50}$ of 12 mmHg.

Thus, (oxy)hemoglobin has been shown to have sufficient affinity for AGATP to form a stable complex. Binding of SFH to AGATP lowers the oxygen affinity of SFH to a level equal to that of the soluble SFH-ATP complex, indicating that complex formation is due to specific binding of SFH to the ATP moiety of the gel.

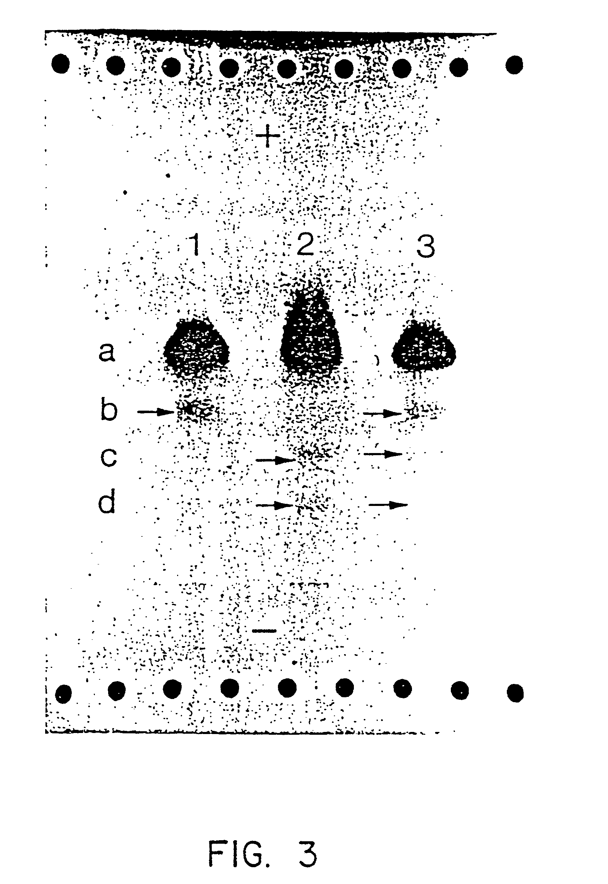

Cellulose acetate electrophoresis of SFH indicates the presence of minor components (Figure 3), two of which are absent from the retained fraction. Two of these minor bands are enhanced in the unretained fraction (Lane 2) while one is enhanced in the retained fraction (Lane 2). Furthermore, there is an increase in hemoglobin with high electrophoretic mobility in the unretained fraction. Thus, polyanion affinity chromatography is shown to improve the purity and quality of SFH.

It will be appreciated that although the Example 1 employs stroma-free hemoglobin as the source of (oxy)hemoglobin, other sources of (oxy)hemoglobin may also be employed, including used blood (from open-heart surgery, for example), human placental extract, and hemoglobin containing solutions produced by biotechnological methods.

## Example 2

Purification of Glyoxylated Hemoglobin by Agarose-ATP Affinity Chromatography

In this example which illustrates the second aspect of the invention, modified glyoxylated hemoglobin (G-Hb) was prepared according to the method of Acharya et al (Fed. Proc., Fed. Amer. Soc. Exp. Biol., Vol. 41, p.1174, 1982). Hemoglobin concentrations were measured using a Corning 2500 CO-oximeter. Agarose-hexane-adenosine-5'-triphosphate (ATP-agarose) (Type 4), containing 9.2 umoles of ATP per ml of gel, was purchased from Pharmacia P-L Biochemicals. Affinity chromatography of hemoglobin solutions was performed using a Pharmacia Fast Protein Liquid Chromatography System. The experimental conditions used are similar to those described in Example 1 above.

In order to determine whether ATP-agarose affinity chromatography is capable of purifying modified hemoglobin, the hemoglobin glyoxylation mixture (G-Hb reaction mixture) was passed through the column and the fractions characterized as in Example 1 above. This produced a similar elution profile (results not shown) to that reported by Hsia et al (J. Chrom., Vol. 303, pp. 425-428, 1984) showing an unretained peak G-Hb-1 and a retained peak G-Hb-II.

The oxygen dissociation curves of the starting solutions and their ATP-agarose fractions showed the following order of $P_{50}$s:

G-Hb-II < G-Hb reaction mixture < G-Hb-I

The $P_{50}$ of G-Hb-I is approximately twice of that of G-Hb-II. This result is, again, similar to those reported by Hsia et al (J. Chrom., Vol. 303, pp. 425-428, 1984). The present result further confirms that the heterogeneity in glyoxylated hemoglobin preparation is due to variation in the extent of glyoxylation. Right shifting of the oxygen dissociation curve is proportional to the extent of glyoxylation. The Agarose-ATP affinity chromatography is thus capable of purifying modified hemoglobin (G-Hb-I) with optimal oxygen affinity.

Thus, this purification technique can be used as a method for the purification of modified hemoglobins in general (e.g. pyridoxal phosphate and ATP modified hemoglobins). The pure modified hemoglobin which results is believed to be a superior starting-material for the preparation of hemoglobin-based blood substitute, because it has optimally right shifted oxygen affinity and it is free of vasoconstrictive unmodified hemoglobin.

## Example 3

Outdated human red blood cells were obtained from the Canadian Red Cross. Stroma-free hemoglobin (SFH) was prepared as in Example 1. Adenosine triphosphate and sodium metaperiodate were purchased from Sigma Chemical Company, St. Louis, Missouri. Periodate oxidized ATP was prepared according to the method of Lowe et al, Biochem. Soc. Trans., Vol. 7 (1979) p.1131. ATP-agarose was prepared by the method of Lamed et al (see Example 1).

ATP-hemoglobin was prepared according to a modified method of Greenburg and Maffuid, "Progress in Clinical and Biological Research" Vol. 122, Advances in Blood Substitute Research, Alan R. Liss, Inc., New York (1983)9. In the modified procedure, 2 ml of caprylic alcohol were added per 100 ml of 7% hemoglobin solution in 5 mM phosphate buffer, pH 7.0. Nitrogen was then bubbled for 2 hours to deoxygenate the hemoglobin solution. Required amount of periodate oxidized ATP in 50 mM Bis-Tris buffer pH 7.0 which had been deoxygenated under nitrogen was then added into the deoxygenated hemoglobin solution to give a molar ratio of 1.2:1 ATP:hemoglobin. Nitrogen was bubbled through the reaction mixture (ATP-hemoglobin solution) for another 90 minutes. The Schiff base of ATP-hemoglobin conjugate was reduced with sodium borohydride (NaBH$_4$) in 1 mM sodium hydroxide (NaOH) at a molar ratio of 20:1 NaBH$_4$/ hemoglobin. Again N$_2$ was bubbled through the mixture for another two hours.

Upon completion of the reduction step, the solution was oxygenated and dialyzed against 50 mM Bis-Tris, pH 7.0. Aliquots of the solution containing approximately 50 mg of hemoglobin were then passed through a column packed with 5 ml of ATP-agarose gel (approximately 30% of column capacity) which had been equilibrated with 50 mM Bis-Tris, pH 7.0. The unretained hemoglobin fraction was eluted by five volumes of the same buffer, and the retained fraction was then eluted by 0.2 M NaCl in 50 mM Bis-Tris, pH 7.0. The fractions were collected, concentrated, and dialyzed against 50 mM Bis-Tris, pH 7.0, with a membrane concentrator, P-Micro ProDicon, Pierce, Rockford, Illinois. The oxygen dissociation curve of each fraction was determined by an Aminco Hem-O-Scan oxygen dissociation analyzer, American Instrument Company, Silver Spring, Maryland. The P$_{50}$ was read directly from the oxygen dissociation curve. The unretained hemoglobin fraction, i.e. the ATP-hemoglobin had a P$_{50}$ of 35, whereas the retained, (oxy)hemoglobin had a P$_{50}$ of 5 mm Hg.

Samples not exceeding 100 ug of hemoglobin in 0.01 M malonate, pH 5.7 (buffer A), were applied to a HR 5/5 MonoS prepacked column (Pharmacia) equilibrated with buffer A. The fractions were eluted with a linear gradient of buffer B (0.3 M LiCl in buffer A) on a Pharmacia LCC 500 FPLC system, Pharmacia, Montreal, Canada.

The elution profile of the products from the column showed the unmodified hemoglobin, with its polyanion binding site intact, is capable of binding to the ATP-agarose and is, therefore, retained on the column while the modified hemoglobin, with one or more ATP molecule(s) covalently occupying its anion-binding site, is eluted as the unretained fraction. The unmodified hemoglobin can be readily recovered for further modification. The results indicated near quantitative separation of the modified and unmodified hemoglobin from the reaction mixture by ATP-agarose affinity chromatography.

## Example 4

Pyridoxal phosphate modified hemoglobin (PyP-Hb) was prepared by the method of F. Devenuto and A. Zegna (J. Surg. Res. 34, pp. 205-212, 1983). The resultant reaction mixture contained about 75% PyP-Hb, the remainder being residual unmodified Hb. The mixture was treated with agarose-ATP gel and the liquid residues physically separated from the solids. The liquid residue was analysed and found to be substantially pure PyP-Hb. The solid gel was treated with ATP, for one batch, and chloride ion, for another batch, as competing anions, and substantially pure hemoglobin recovered therefrom.

## Claims

1. A method for the isolation of (oxy)hemoglobin from (oxy)hemoglobin solutions or mixtures, comprising:

(a) providing a chromatographic gel which has immobilized thereon a polyanion which specifically binds hemoglobin via its polyanion binding site, the polyanion being chemically linked to the chromatographic gel by a hydrophobic chemical spacer group;

(b) passing the (oxy)hemoglobin-containing solution or mixture through the gel, whereby the (oxy)hemoglobin is retained in the gel selectively, while the other components pass therethrough; and

(c) introducing into the chromatographic gel an anion which competes with the polyanion originally in the gel, whereby (oxy)hemoglobin is eluted from the gel.

2. A method according to claim 1 wherein the polyanion is selected from the group consisting of a nucleoside triphosphate, a diphosphoglycerate, an inositol phosphate and an inositol sulphate.

3. A method according to claim 1 or claim 2 wherein the chromatographic gel is selected from the group consisting of cross linked polysaccharide gels and silica gel.

4. A method according to any preceding claim wherein the hydrophobic chemical spacer group is derived from adipic acid, diaminohexane and adipic acid dihydrazide.

5. A method according to claim 2 wherein the polyanion is adenosine triphosphate or inositol tetra- or pentaphosphate.

6. A method according to claim 3 wherein the gel is agarose gel.

7. A method according to claim 4 wherein the chemical spacer group is adipic acid.

8. A method according to any preceding claim wherein the competing anion for the elution step is selected from inositol hexaphosphate, adenosine triphosphate, pyridoxal phosphate, diphosphoglycerate, adenosine diphosphate, phosphate ion and chloride ion.

9. A method according to claim 1 wherein the source of (oxy)hemoglobin is stroma-free hemoglobin.

10. A method according to claim 9 wherein the elution anion and the stroma-free hemoglobin both include a suitable buffer, which does not interfere with the binding of hemoglobin to the polyanion.

11. A method according to any preceding claim wherein the (oxy)hemoglobin mixture or solution which is subjected to the isolation procedure is a liquid reaction mixture containing modified and unmodified hemoglobin.

12. A method according to claim 11 wherein the polyanion/chromatographic gel complex is ATP-agarose gel.

13. A method according to claim 11 or claim 12 wherein the elution anion is selected from inositol hexaphosphate, adenosine triphosphate, pyridoxal phosphate, diphosphoglycerate, adenosine diphosphate, phosphate ion and chloride ion.

14. A method according to claim 11 wherein the modified hemoglobin is glyoxylated hemoglobin.

15. A method according to claim 11 wherein the modified hemoglobin is ATP-hemoglobin.

16. A method according to claim 11 wherein the modified hemoglobin is pyridoxal phosphate-hemoglobin.

## Patentansprüche

1. Verfahren zur Isolierung von (Oxy)Hämoglobin aus (Oxy)Hämoglobinlösungen oder -mischungen mit den Schritten
(a) Bereitstellung eines chromatografischen Gels mit einem auf diesem fixierten Polyanion, welches über seine Polyanionbindungsstelle spezifisch Hämoglobin bindet, wobei das Polyanion an das chromatografische Gel durch eine hydrophobe chemische Spacergruppe chemisch gebunden ist,
(b) Durchleiten der (Oxy)Hämoglobinlösung oder -mischung durch das Gel, wobei das (Oxy)Hämoglobin selektiv in dem Gel zurückgehalten wird, während die anderen Komponenten durchlaufen; und
(c) Einführung eines Anions in das chromatografische Gel, das mit dem ursprünglich in dem Gel vorhandenen Polyanion konkurriert, wodurch das (Oxy)Hämoglobin aus dem Gel eluiert wird.

2. Verfahren nach Anspruch 1, bei dem das Polyanion aus der von einem Nucleosidtriphosphat, einem Diphosphoglycerat, einem Inositolphosphat und einem Inositolsulfat gebildeten Gruppe ausgewählt ist.

3. Verfahren nach Anspruch 1 oder Anspruch 2, bei dem das chromatografische Gel aus der von vernetzten Polysaccharidgelen und Silicagel gebildeten Gruppe ausgewählt ist.

4. Verfahren gemäß einem jeden der vorhergehenden Ansprüche, bei dem die hydrophobe chemische Spacergruppe von Adipinsäure, Diaminohexan und Adipinsäuredihydrazid abgeleitet ist.

5. Verfahren nach Anspruch 2, bei dem das Polyanion Adenosintriphosphat oder Inositoltetra- oder -pentaphosphat ist.

6. Verfahren nach Anspruch 3, bei dem das Gel Agarosegel ist.

7. Verfahren nach Anspruch 4, bei dem die chemische Spacer-gruppe Adipinsäure ist.

8. Verfahren gemäß einem jeden der vorhergehenden Ansprüche, bei dem das konkurrierende Anion für den Elutionsschritt aus Inositolhexaphosphat, Adenosintriphosphat, Pyridoxalphosphat, Diphosphoglycerat, Adenosindiphosphat, Phosphationen und Chloridionen ausgewählt ist.

9. Verfahren nach Anspruch 1, bei dem die (Oxy)Hämoglobinquelle stromafreies Hämoglobin ist.

10. Verfahren nach Anspruch 9, bei dem das Elutionsanion und das stromafreie Hämoglobin jeweils einen geeigneten Puffer enthalten, der die Bindung des Hämoglobins an das Polyanion nicht stört.

11. Verfahren gemäß einem jeden der vorhergehenden Ansprüche, bei dem die (Oxy)Hämoglobinmischung oder -lösung, die dem Isolierungsverfahren unterworfen wird, ein flüssiges, modifiziertes und unmodifiziertes Hämoglobin enthaltendes Reaktionsgemisch ist.

12. Verfahren nach Anspruch 11, bei dem der Polyanion/chromatografische Gel-Komplex ATP-Agarose-Gel ist.

13. Verfahren nach Anspruch 11 oder Anspruch 12, bei dem das Elutionsanion aus Inositolhexaphosphat, Adenosintriphosphat, Pyridoxalphosphat, Diphosphoglycerat, Adenosindiphosphat, Phosphationen und Chloridionen ausgewählt ist.

14. Verfahren nach Anspruch 11, bei dem das modifizierte Hämoglobin glyoxyliertes Hämoglobin ist.

15. Verfahren nach Anspruch 11, bei dem das modifizierte Hämoglobin ATP-Hämoglobin ist.

16. Verfahren nach Anspruch 11, bei dem das modifizierte Hämoglobin Pyridoxalphosphat-Hämoglobin ist.

## Revendications

1. Procédé pour l'isolement de l'(oxy)hémoglobine de solutions ou mélanges d'(oxy)hémoglobine, comprenant les étapes suivantes:
(a) on part d'un gel chromatographique portant un polyanion immobilisé qui fixe spécifiquement l'hémoglobine via son site de fixation du polyanion, le polyanion étant chimiquement lié au gel chromatographique par un groupe écarteur chimique hydrophobe;

(b) on fait passer la solution ou le mélange d'(oxy)hémoglobine à travers le gel, l'(oxy)hémoglobine étant ainsi retenue sélectivement dans le gel, tandis que les autres composants traversent celui-ci; et

(c) on introduit dans le gel chromatographique un anion qui entre en compétition avec le polyanion initialement dans le gel, l'(oxy)hémoglobine étant ainsi éluée du gel.

2. Un procédé selon la revendication 1, dans lequel le polyanion est choisi parmi un mucléoside-triphosphate, un diphosphoglycérate, un phosphate d'inositol et un sulfate d'inositol.

3. Un procédé selon la revendication 1 ou 2, dans lequel le gel chromatographique est choisi parmi les gels de polysaccharides réticulés et le gel de silice.

4. Un procédé selon l'une quelconque des revendications précédentes, dans lequel le groupe écarteur chimique hydrophobe est dérivé de l'acide adipique, du diaminohexane et du dihydrazide d'acide adipique.

5. Un procédé selon la revendication 2, dans lequel le polyanion est l'adénosine-triphosphate ou l'inositol-tétra- ou penta-phosphate.

6. Un procédé selon la revendication 3, dans lequel le gel est un gel d'agarose.

7. Un procédé selon la revendication 4, dans lequel le groupe écarteur chimique est l'acide adipique.

8. Un procédé selon l'une quelconque des revendications précédentes, dans lequel l'anion en compétition pour l'étape d'élution est choisi parmi l'inositol-hexaphosphate, l'adénosine-triphosphate, le pyridoxal-phosphate, le diphosphoglycérate, l'adénosine-diphosphate, l'ion phosphate et l'ion chlorure.

9. Un procédé selon la revendication 1, dans lequel la source d'(oxy)hémoglobine est l'hémoglobine exempte de stroma.

10. Un procédé selon la revendication 9, dans lequel l'anion d'élution et l'hémoglobine exempte de stroma contiennent tous deux un tampon approprié qui ne perturbe pas la fixation de l'hémoglobine au polyanion.

11. Procédé selon l'une quelconque des revendications précédentes, dans lequel le mélange ou solution d'(oxy)hémoglobine que l'on soumet au procédé d'isolement est un mélange réactionnel liquide contenant de l'hémoglobine modifiée ou non.

12. Un procédé selon la revendication 11, dans lequel le complexe polyanion/gel chromatographique est l'ATP-gel d'agarose.

13. Un procédé selon la revendication 11 ou 12, dans lequel l'anion d'élution est choisi parmi l'inositol-hexaphosphate, l'adénosine-triphosphate, le pyridoxal-phosphate, le diphosphoglycérate, l'adénosine-diphosphate, l'ion phosphate et l'ion chlorure.

14. Un procédé selon la revendication 11, dans lequel l'hémoglobine modifiée est l'hémoglobine glyoxylée.

15. Un procédé selon la revendication 11, dans lequel l'hémoglobine modifiée est l'ATP-hémoglobine.

16. Un procédé selon la revendication 11, dans lequel l'hémoglobine modifiée est le pyridoxal-phosphate-hémoglobine.

FIG. 1

FIG. 2

FIG. 3